# FASCICULE DE BREVET EUROPEEN

(11) **EP 4 295 882 B1**
(45) Date de publication et mention de la délivrance du brevet: **11.12.2024**
(21) Numéro de dépôt: 23178092.5
(22) Date de dépôt: 07.06.2023
(51) Int. Cl.: A61M 16/00, A61M 16/12

(54) **AFFICHAGE DE LA DOSE DE NO PAR UN DISPOSITIF DE FOURNITURE DE NO EN PHASE DE PAUSE**
ANZEIGE DER NO-DOSIS DURCH EINE VORRICHTUNG ZUR BEREITSTELLUNG VON NO IN PAUSENPHASE
DISPLAY OF NO DOSE BY NO DELIVERY DEVICE IN PAUSE PHASE

(30) Priorité: 24.06.2022 FR 2206335
(43) Date de publication de la demande: 27.12.2023
(73) Titulaire: Inosystems, 92160 Antony (FR)
(72) Inventeur: BLANDIN, Yann, 92220 Bagneux (FR); SCHMITT, Mary, 92220 Bagneux (FR); PROUVEZ, Nathan, 92160 Antony (FR); MARCHAL, Frederic, 92160 Antony (FR)
(74) Mandataire: Air Liquide

(56) Documents cités:
- EP-A1- 3 308 820
- EP-A1- 3 906 954
- WO-A1-2013/070712
- US-A1- 2015 320 951
- US-A1- 2022 106 189

## Description

L'invention concerne un dispositif ou appareil de fourniture de monoxyde d'azote (NO) gazeux, en particulier d'un mélange de NO et d'azote (N₂), et une installation d'administration de NO à un patient comprenant un tel dispositif de fourniture de NO.

Le monoxyde d'azote inhalé ou NOi est un médicament gazeux couramment utilisé pour traiter des patients souffrant d'hypertension artérielle pulmonaire aiguë, en particulier les vasoconstrictions pulmonaires chez l'adulte ou l'enfant, y compris les nouveau-nés (PPHN), comme décrit par exemple par EP-A-560928 ou EP-A-1516639.

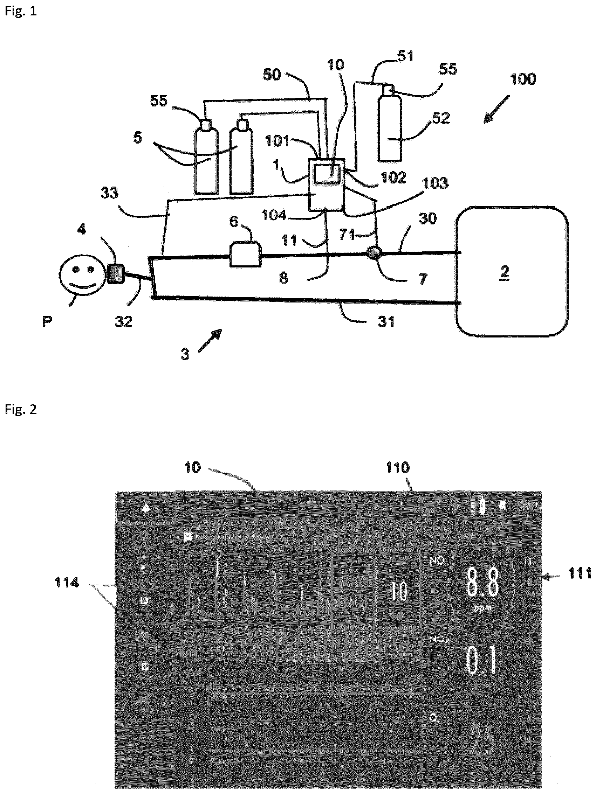

Une installation de mise en oeuvre d'un traitement par NOi, couramment appelée installation d'administration de NO, comprend classiquement une ou des bouteilles de mélange NO/N₂ alimentant un dispositif de fourniture de NO qui délivre le mélange NO/N₂ à débit contrôlé, un appareil d'assistance respiratoire, aussi appelé ventilateur médical, pour fournir un gaz respiratoire contenant au moins 21 % vol. d'oxygène, tel un mélange O₂/N₂ ou de l'air, auquel est ajouté le NO (i.e. le mélange NO/N₂) fourni par le dispositif de fourniture de NO, des éléments de circuit, par exemple un ou plusieurs conduits flexibles, pour acheminer les flux gazeux entre ces différents équipements et jusqu'au patient, et une interface respiratoire, telle une sonde trachéale, pour fournir le mélange gazeux contenant le NO au patient. On peut prévoir aussi un humidificateur de gaz pour humidifier le mélange gazeux avant son administration au patient. Une telle installation est schématisée en Fig. 1.

Habituellement, le mélange NO/N₂ délivré par le dispositif de fourniture de NO est injecté dans le flux respiratoire contenant au moins 21% vol. d'oxygène (i.e. air ou mélange O₂/N₂) provenant du ventilateur médical avant d'être administré par inhalation au patient sous forme d'un mélange respiratoire final (i.e. mélange NO/N₂/O₂ ou NO/N₂/air) contenant généralement quelques dizaines ppmv de NO (ppm en volume) et au moins 21% vol. d'oxygène O₂, par exemple de l'ordre de 1 à 80 ppmv de NO, le reste étant essentiellement de l'azote (N₂).

Une telle installation d'administration de NO est utilisée en milieu hospitalier pour administrer le traitement par NOi et ainsi soigner les patients ayant besoin d'inhaler du NO pour traiter leur hypertension artérielle pulmonaire. Des exemples de telles installations d'administration de NO sont donnés par les documents EP3906954A1, WO-A-2012/094008, US-A-2015/320951, US-A-2015/273175, JP-A-H11192303, WO-A-02/40914 et US-A-2003/116159.

En particulier, le médicament à base de NO est indiqué dans le traitement des patients atteints d'une crise aiguë d'hypertension artérielle pulmonaire, qui est une situation clinique sévère pendant laquelle le pronostic vital des patients est engagé.

En cours du traitement par NOi, le NO doit être fourni selon une posologie précise et préférentiellement de manière interrompue entre le début et la fin du traitement par NOi.

Cependant, pendant le déroulé du traitement par NOi, des mises en pause du système d'administration peuvent être nécessaires, voire indispensables, par exemple lorsque le patient est temporairement déconnecté du système de ventilation afin de subir d'autres soins médicaux, telles des aspirations bronchiques ou autres.

Aujourd'hui, les dispositifs d'administration du NO inhalé connus affichent la dose de NO en cours d'administration, c'est-à-dire la dose de NO désirée ayant été choisie par le personnel soignant et délivrée pendant la durée du traitement à proprement parler.

Or, une mise en pause de ces dispositifs d'administration du NO connus requiert une mise à zéro de la dose de NO devant être administrée au patient, à défaut de quoi, du NO continue à être fourni par le dispositif de fourniture de NO pendant toute la durée de la pause, typiquement plusieurs minutes, voire dizaines de minutes, ce qui ne serait évidemment pas souhaitable.

Ensuite, lors de la reprise du traitement, le personnel médical doit à nouveau indiquer au dispositif d'administration du NO, la dose de NO désirée devant être administrée pendant le reste du traitement. Or, cette manière de procéder n'est pas satisfaisante car elle engendre des risques pour les patients. En effet, l'équipe médicale peut commettre une erreur de réglage ou même de prescription si elle a oublié la dose réglée avant la mise en pause du dispositif d'administration du NO.

Les patients sont alors exposés au risque de recevoir une dose de NO non-conforme à la prescription, lors de la reprise de l'administration de NO, c'est-à-dire à la fin de la mise en pause.

Un problème est dès lors de pouvoir éviter ou limiter les erreurs de réglage ou de prescription de NO par le personnel soignant, lors de la reprise d'un traitement par NO ayant été mis en pause pendant typiquement plusieurs minutes, voire dizaines de minutes, de manière à diminuer les risques pour le patient de se voir administrer une dose de NO non-conforme à sa prescription.

Une solution de l'invention concerne alors un dispositif de fourniture de NO, comprenant :
- un passage interne pour acheminer un flux gazeux contenant du NO, typiquement un mélange gazeux NO/N₂,
- des moyens à valve pour contrôler le flux gazeux dans le passage interne,
- des moyens de pilotage pour piloter au moins les moyens à valve,
- des moyens de sélection de dose configurés pour permettre à un utilisateur de choisir ou d'ajuster une dose de NO à administrer,
- des moyens de mémorisation configurés pour mémoriser au moins ladite dose de NO à administrer,
- des moyens de démarrage de traitement actionnables par l'utilisateur et coopérant avec les moyens de pilotage pour débuter ou reprendre un traitement par NO,
- des moyens d'arrêt de traitement actionnables par l'utilisateur et coopérant avec les moyens de pilotage pour mettre en pause ou stopper définitivement un traitement par NO, et
- un afficheur graphique configuré pour afficher la dose de NO à administrer avant le début du traitement par NO ou la dose administrée au patient pendant le traitement par NO.

Selon l'invention, l'afficheur graphique est en outre configuré pour continuer à afficher la dose de NO ayant été administrée au patient, pendant une période de pause (dt) durant laquelle le traitement par NO est interrompu temporairement, c'est-à-dire une pendant une mise en pause, en réponse à un actionnement par l'utilisateur des moyens d'arrêt de traitement.

Selon le mode de réalisation considéré, le dispositif de l'invention peut comprendre l'une ou plusieurs des caractéristiques suivantes :
- les moyens de pilotage sont en outre configurés pour piloter les moyens à valve pour fournir un débit gazeux correspondant à la dose de NO à administrer pendant le traitement par NO, en réponse à l'actionnement par l'utilisateur des moyens de démarrage de traitement.
- les moyens de pilotage sont en outre configurés pour piloter les moyens à valve pour interrompre temporairement, pendant la période de pause (dt), tout débit gazeux en réponse à l'actionnement par l'utilisateur des moyens d'arrêt de traitement.
- les moyens de pilotage sont configurés pour commander l'afficheur graphique pour afficher la dose de NO à administrer, avant et pendant le traitement par NO et pendant la période de pause (dt) durant laquelle le traitement par NO est interrompu temporairement.
- les moyens de pilotage sont configurés pour commander l'afficheur graphique pour continuer à afficher, pendant la période de pause (dt), la dose de NO ayant été administrée au patient juste avant (i.e. immédiatement avant) actionnement par l'utilisateur des moyens d'arrêt de traitement, c'est-à-dire avant la mise en pause.
- les moyens de pilotage sont configurés pour commander l'afficheur graphique pour afficher une dose ajustée de NO à administrer au patient, après la période de pause (dt), ladite dose ajustée de NO étant choisie ou ajusté par l'utilisateur, pendant la période de pause (d), via les moyens de sélection de dose.
- la période de pause (dt) a une durée inférieure ou égale à 30 minutes, de préférence comprise entre 1 et 20 minutes environ.
- les moyens de mémorisation sont configurés pour mémoriser la dernière dose de NO administrée au patient immédiatement avant le début de la période de pause (dt), c'est-à-dire avant mise en pause.
- les moyens de démarrage de traitement et les moyens d'arrêt de traitement comprennent un unique et même organe d'actionnement, actionnable par l'utilisateur.
- alternativement, les moyens de démarrage de traitement et les moyens d'arrêt de traitement comprennent des organes d'actionnement distincts, actionnables par l'utilisateur.
- le ou les organes d'actionnement comprennent une ou plusieurs touches, boutons, sélecteurs rotatifs, curseurs ou analogues.
- le ou les organes d'actionnement comprennent une ou plusieurs touches de sélection virtuelles affichées sur l'afficheur graphique.
- l'afficheur graphique est configuré pour afficher une ou plusieurs touches virtuelles.
- l'afficheur graphique est un écran d'affichage numérique à commande tactile.
- les moyens de pilotage comprennent au moins un (micro)processeur.
- les moyens de sélection de dose comprennent au moins une touche de sélection tactile affichée sur l'écran d'affichage.
- l'écran d'affichage est configuré pour afficher ladite au moins une touche de sélection tactile, de préférence plusieurs touches de sélection tactiles.
- la ou les touches de sélection tactile sont configurées pour permettre d'opérer un choix parmi plusieurs teneurs en NO proposées ou alternativement, comprennent des touches « + » et « - » permettant d'incrémenter ou de décrémenter une valeur de NO donnée par palier, par exemple de 0.1 ppmv ou 1 ppmv, ou autre (e.g. 2 en 2 ppmv, 3 en 3 ppmv... ou 5 en 5 ppmv..).
- ladite teneur en NO désirée qui est choisie ou sélectionnée par l'utilisateur par action sur ladite touche de sélection tactile est fournie aux moyens de pilotage, de préférence par action digitale.
- la sélection ou la fixation de la dose de NO désirée se fait par appui digital de la part de l'utilisateur sur la ou les touches de sélection à actionnement tactile affichées sur l'écran d'affichage.
- les moyens de sélection de dose de NO sont configurés pour permettre à l'utilisateur de fixer ou sélectionner une teneur en NO désirée comprise entre 0,1 et 80 ppmv.
- l'écran d'affichage est à affichage en couleurs ou en noir et blanc.
- les moyens de pilotage sont configurés pour commander le ou les affichages sur l'afficheur graphique, i.e. l'écran d'affichage d'informations.
- les moyens de pilotage comprennent un ou plusieurs (micro)processeurs, par exemple un microcontrôleur.
- les moyens de pilotage comprennent au moins une carte électronique comprenant ledit au moins un microprocesseur.
- les moyens de pilotage comprennent au moins un (micro)processeur mettant en oeuvre au moins un algorithme, par exemple de traitement de données, de calcul ou autre.
- la ou les touches virtuelles affichées sur l'afficheur graphique sont des touches tactiles.
- l'afficheur graphique est à dalle tactile.

Selon un autre aspect, l'invention concerne aussi une installation d'administration de gaz thérapeutique contenant du NO, à un patient (P) comprenant un dispositif de fourniture de NO selon l'invention, en particulier tel que décrit ci-dessus, alimenté en mélange NO/N₂ par au moins un récipient de gaz sous pression et en oxygène par un récipient d'oxygène sous pression, ledit dispositif de fourniture de NO fournissant un mélange NO/N₂ à un circuit de gaz respiratoire raccordé à un ventilateur médical fournissant un mélange O₂/N₂ ou de l'air.

Selon le mode de réalisation considéré, l'installation d'administration de gaz thérapeutique de l'invention peut comprendre l'une ou plusieurs des caractéristiques suivantes :
- le dispositif de fourniture de NO peut comprendre une sortie principale fournissant le mélange NO/N₂ au circuit de gaz respiratoire raccordé au ventilateur médical, de préférence via un conduit d'injection de NO ou ligne d'injection de NO, c'est-à-dire un tuyau flexible ou analogue.
- ledit au moins un récipient de gaz sous pression contient le mélange NO/N₂.
- ledit au moins un récipient de gaz sous pression contient un mélange NO/N₂ contenant de 100 à 1000 ppmv de NO, le reste étant de l'azote.
- le récipient d'oxygène sous pression contient de l'oxygène médical.
- le ou les récipients sont des bouteilles de gaz.
- le ou les récipients de gaz contiennent un mélange NO/N₂ ou de l'oxygène médical à une pression d'au moins 150 bar, voire au moins 180 bar.
- le circuit de gaz respiratoire comprend (au moins) une branche inspiratoire et une branche expiratoire.
- la branche inspiratoire et la branche expiratoire sont reliées fluidiquement l'une à l'autre via une pièce de jonction, telle une pièce en Y.
- la pièce de jonction est raccordée fluidiquement à une interface respiratoire, telle une sonde trachéale, un masque respiratoire ou des lunettes pour oxygénation.
- la branche inspiratoire et la branche expiratoire comprennent des tuyaux flexibles.
- un humidificateur de gaz est agencé sur le circuit de gaz respiratoire, en particulier sur la branche inspiratoire.
- la branche inspiratoire comprend un capteur de débit relié électriquement et/ou via des tubulures au dispositif de fourniture de NO, en particulier aux moyens de pilotage.
- le capteur de débit est agencé sur la branche inspiratoire en amont du site d'injection de NO.
- le capteur de débit est du type massique ou à différentiel de pression.
- une ligne de prélèvement de gaz relient fluidiquement le dispositif de fourniture de NO au circuit de gaz respiratoire, de préférence à proximité de la pièce de jonction, i.e. la pièce en Y.
- le ventilateur médical et le dispositif de fourniture de NO sont alimentés électriquement par au moins une source de courant électrique.
- le dispositif de fourniture de NO peut comprendre une sortie de secours, i.e. une sortie secondaire, par exemple un port ou un orifice, pouvant être raccordée fluidiquement à un ballon d'insufflation manuel.

D'une façon générale, dans le cadre de l'invention, les termes « moyens de » ou « moyens à » sont considérés comme équivalents et substituables aux termes « dispositif de », « dispositif à » ou analogue. Par exemple, les termes « moyens à valve » sont considérés comme équivalents et substituables aux termes « dispositif à valve », les termes « moyens de pilotage » sont considérés comme équivalents et substituables aux termes « dispositif de pilotage », etc...

La présente divulgation concerne aussi une méthode (non revendiquée) de traitement thérapeutique d'un patient, dans laquelle on met en oeuvre un dispositif de fourniture de NO selon l'invention et/ou une installation d'administration de gaz thérapeutique contenant du NO à un patient (P), comprenant un dispositif de fourniture de NO selon l'invention, en particulier tels que décrits ci-dessus, pour administrer par inhalation un gaz thérapeutique contenant du monoxyde d'azote ou NO (i.e. un médicament gazeux) au patient à traiter, ledit patient souffrant d'hypertension artérielle pulmonaire, en particulier de vasoconstrictions pulmonaires.

Selon le mode de réalisation considéré, la méthode de traitement peut comprendre l'une ou plusieurs des caractéristiques suivantes :
- le patient est un adulte, un adolescent, un enfant, un nouveau-né ou un bébé.
- le patient souffre d'une hypertension artérielle pulmonaire aiguë.
- le patient souffre d'une hypertension pulmonaire persistante du nouveau-né (PPHN ou *Persistent Pulmonary Hypertension of the Newborn*)*.*
- l'hypertension pulmonaire est péri-opérative et associée à une intervention de chirurgie cardiaque.
- le gaz thérapeutique contenant du monoxyde d'azote (NO) est administré à une teneur inférieure à 40 ppmV.
- le gaz thérapeutique contient du monoxyde d'azote (NO), de l'azote et de l'oxygène (au moins 21 %vol. environ), et des impuretés éventuelles.
- le gaz thérapeutique contenant du monoxyde d'azote (NO) est administré via une sonde d'intubation trachéale ou analogue.
- le dispositif de fourniture de NO est alimenté par au moins un récipient de gaz sous pression, e.g. une bouteille de gaz, contenant un mélange NO/N₂ contenant de 100 à 1000 ppmv de NO, le reste étant de l'azote.
- l'installation d'administration de gaz comprend un ventilateur médical fournissant un gaz contenant de l'oxygène (au moins 21 %vol. environ), par exemple un mélange O₂/N₂ ou de l'air.

L'invention va maintenant être mieux comprise grâce à la description détaillée suivante, faite à titre illustratif mais non limitatif, en référence aux figures annexées parmi lesquelles :
Fig. 1 représente un mode de réalisation d'une installation d'administration de gaz thérapeutique à un patient incorporant un dispositif de fourniture de NO selon l'invention,
Fig. 2 représente un mode de réalisation de l'afficheur graphique d'un dispositif de fourniture de NO selon l'invention montrant les informations affichées pendant un traitement par NO, par exemple au moyen de l'installation de Fig. 1, et
Fig. 3 représente un mode de réalisation de l'afficheur graphique d'un dispositif de fourniture de NO selon l'invention montrant les informations affichées pendant une période de pause durant laquelle le traitement par NO est interrompu temporairement.

Fig. 1 schématise un mode de réalisation d'une installation 100 d'administration de gaz thérapeutique, i.e. un mélange gazeux à base de NO, typiquement un mélange NO/O₂/N₂, à un patient P incorporant le dispositif de fourniture de NO 1 selon la présente invention.

Plus précisément, elle comprend ici deux récipients de gaz sous pression 5, agencés en parallèle, contenant chacun un mélange gazeux de NO et d'azote (N₂), i.e. un mélange NO/N₂, contenant typiquement de 250 à 1000 ppmv de NO et de l'azote (N₂) pour le reste conditionné à une pression pouvant atteindre 180 bar ou plus, par exemple un mélange NO/N₂, contenant 450 ppmv ou 800 ppmv de NO. De tels récipients de gaz 5 sont couramment appelés bouteilles de NO 5.

Les bouteilles de NO 5 fournissent le mélange NO/N₂ à au dispositif de fourniture de NO 1 selon l'invention. Elles sont reliées fluidiquement au dispositif de fourniture de gaz 1 par des lignes 50 de fourniture de NO, i.e. des canalisations de gaz, tels des tuyaux flexibles ou analogue. Chaque ligne 50 de fourniture de NO est reliée à un port d'entrée de NO 101 du dispositif de fourniture de NO 1 pour alimenter un circuit principal de gaz 200 interne au boîtier 103 du dispositif de fourniture de NO 1.

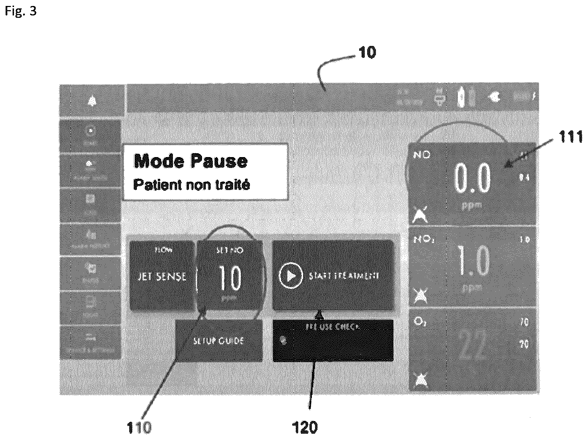

Le boitier 103 du dispositif de fourniture de NO 1 comprend aussi un afficheur graphique 10, tel un écran tactile, configuré pour afficher diverses informations, notamment les doses de NO, comme expliqué ci-après et illustré en Fig. 2 et Fig. 3.

Le dispositif de fourniture de NO 1 comprend aussi un port d'entrée d'oxygène 102 relié fluidiquement, via une ligne d'amenée d'oxygène 51, tel un tuyau flexible ou analogue, à une source d'oxygène par exemple un récipient d'oxygène sous pression 52, typiquement une bouteille d'O₂ ou, alternativement, le réseau hospitalier, i.e. une canalisation d'amenée d'oxygène agencée dans le bâtiment hospitalier où est soigné le patient P.

Les bouteilles de NO 5 et la bouteille d'O₂ 52 sont équipées d'un robinet de distribution de gaz 55, de préférence intégrant des moyens de détente de gaz, c'est-à-dire un RDI ou robinet à détendeur intégré, de manière à pouvoir contrôler le débit et/ou la pression du gaz qu'elles délivrent. Le robinet de distribution de gaz 55 est préférentiellement protégé contre les chocs par un capotage de protection.

Par ailleurs, l'installation 100 comprend aussi un ventilateur médical 2, c'est-à-dire un appareil d'assistance respiratoire, fournissant un flux de gaz respiratoire contenant au moins 21% vol. d'oxygène, tel de l'air ou un mélange oxygène/azote (N₂/O₂), au patient P.

Le ventilateur médical 2 est fluidiquement relié au patient P via un circuit de gaz 3 respiratoire qui est ici à deux branches respiratoires 30, 31 étant donné qu'il comprend une branche inspiratoire 30, c'est-à-dire une ligne d'alimentation en gaz, servant à amener le gaz respiratoire au patient P et une branche expiratoire 31 sevrant à récupérer le gaz enrichi en CO₂ expiré par le patient P.

Les deux branches respiratoires 30, 31 sont typiquement des tuyaux flexibles en polymère ou analogue. Les deux branches respiratoires 30, 31 sont, d'une part, raccordées au ventilateur médical 2 et, d'autre part, reliées l'une à l'autre au niveau d'une pièce de jonction 32, typiquement une pièce en Y, laquelle est en communication fluidique avec une interface respiratoire 4 fournissant le gaz au patient P, telle une sonde trachéale ou autre.

Bien entendu, le ventilateur médical 2 et le dispositif de fourniture de NO 1 sont normalement alimentés électriquement par une (des) source de courant électrique, en particulier leurs composants nécessitant de l'énergie électrique pour fonctionner, en particulier les moyens de pilotage du dispositif de fourniture de NO 1 et le système de commande du ventilateur médical 2, i.e. carte électronique à microprocesseur(s), ou tout autre composant, notamment la turbine interne motorisée qui fournit le flux d'air ou analogue, i.e. le gaz respiratoire. La source de courant électrique peut être le secteur (110/220V) et/ou une batterie électrique, de préférence rechargeable.

Comme on le voit, le dispositif de fourniture de NO 1 permet d'injecter le mélange NO/N₂ dans la branche inspiratoire 30, via un conduit d'injection de NO 11 débouchant dans la branche inspiratoire 30 en un site d'injection 8, de manière à y opérer un mélange du flux de NO/N₂ et du flux de gaz respiratoire contenant au moins 21% d'O₂, i.e. air ou de mélange oxygène/azote, délivré par le ventilateur médical 2.

Le dispositif de fourniture de NO 1 comprend un orifice de sortie principal 104 situé au débouché de son circuit principal de gaz par lequel le flux de NO/N₂ sort du boitier 103 du dispositif de fourniture de NO 1 et pénètre dans le conduit d'injection de NO 11. Le conduit d'injection de NO 11 est raccordé fluidiquement à l'orifice de sortie principal 104, par exemple via un connecteur ou analogue.

Le mélange gazeux thérapeutique obtenu contient donc de l'oxygène (>21% vol.), de l'azote et une concentration de NO variable et ajustable, typiquement comprise entre 1 et 80 ppmv, du fait de la dilution de produisant lors du mélange des flux gazeux. Bien entendu, des impuretés inévitables peuvent se trouver dans le gaz mais elles ne sont pas souhaitées, en particulier quand le flux de gaz provenant du ventilateur 2 est de l'air atmosphérique plutôt qu'un mélange O₂/N₂.

Avantageusement, on prévoit en outre un humidificateur de gaz 6 agencé ici sur la branche inspiratoire 30 en aval du site d'injection 8 servant à humidifier le flux de gaz thérapeutique, e.g. mélange NO/N₂/O₂, par addition de vapeur d'eau, avant qu'il ne soit inhalé par le patient P, ce qui permet d'éviter ou limiter l'assèchement des voies respiratoires du patient P pendant son traitement par inhalation du gaz.

Selon un autre mode de réalisation, l'humidificateur de gaz 6 pourrait aussi être agencé en amont du site d'injection 8.

Selon le cas, la branche expiratoire 31 servant à recueillir les gaz expirés riches en CO₂ peut comprendre un ou d'autres composants optionnels, comme par exemple un dispositif d'élimination du CO₂, i.e. un piège à CO₂, tel un bac à chaud ou autre, permettant d'éliminer le CO₂ présent dans les gaz expirés par le patient, un filtre ou autre.

Il est aussi prévu sur la branche inspiratoire 30, en amont du site d'injection 8, un capteur de débit 7, par exemple du type massique ou à différentiel de pression, relié au dispositif de fourniture de NO 1, notamment aux moyens de pilotage dudit dispositif de fourniture de NO 1, via une ligne de mesure de débit 71 de gaz respiratoire servant à mesurer le débit de gaz provenant du ventilateur 2 au sein de la branche inspiratoire 30.

Déterminer ce débit du ventilateur permet notamment de réguler le passage du NO au travers du dispositif de fourniture de NO 1, en particulier de pouvoir choisir le débit de mélange NO/N₂ à injecter en fonction de la teneur en NO désirée, de la composition du mélange NO/N₂ provenant des bouteilles et du débit de gaz (i.e. air ou air/O₂) provenant du ventilateur 2.

Par ailleurs, on peut prévoir aussi une ligne de prélèvement 33 de gaz reliant fluidiquement le dispositif de fourniture de NO 1 au circuit de gaz respiratoire 3, de préférence à proximité de la pièce en Y 32, par exemple à environ 10 à 20 cm en amont de la pièce en Y 32, servant à prélever des échantillons de gaz et à vérifier, au moyen d'un analyseur de gaz ou analogue, leur conformité avec le mélange gazeux souhaité devant être administré au patient P.

Plus précisément, le dispositif de fourniture 1 de NO comprend un circuit principal de gaz interne pour acheminer le mélange NO/N₂ entrant par le ou les ports d'entrée de gaz 101 jusqu'au conduit d'injection de NO 11. Ce circuit principal de gaz comprend des moyens de contrôle de débit de NO/N₂, à savoir des moyens à valve, telles des valves, des orifices calibrés..., pilotés par les moyens de pilotage du dispositif de fourniture 1 de NO, typiquement un (ou des) microprocesseur agencé sur une carte électronique, dont le fonctionnement est expliqué ci-après. Tous ces composants sont agencés dans le boitier 103, c'est-à-dire une carcasse externe rigide.

Les moyens de pilotage du dispositif de fourniture 1 de NO commandent aussi les affichages sur l'afficheur 10, tel un écran tactile, de préférence en couleurs.

De plus, le dispositif de fourniture 1 de NO peut aussi comprendre un circuit de secours, appelé circuit de « backup », conçu pour délivrer un débit d'O₂ réglable et un débit fixe de NO afin de pouvoir assurer une fourniture de NO, même en cas de panne ou autre.

Conformément à la présente invention, le dispositif de fourniture de NO 1 comprend en outre des moyens de sélection de dose, telle une touche virtuelle affichée sur l'afficheur 10, configurés pour permettre à un utilisateur de choisir ou d'ajuster une dose de NO à administrer, et des moyens de mémorisation configurés pour mémoriser la dose de NO à administrer, voire d'autres informations.

De plus, il comprend aussi des moyens de démarrage de traitement 120 actionnables par l'utilisateur, telle une touche virtuelle affichée sur l'afficheur 10 comme visible sur Fig. 3, qui coopèrent avec les moyens de pilotage pour débuter ou reprendre un traitement par NO en commandant les moyens à valves pour fournir la dose de NO désirée.

Par analogie, il comprend aussi des moyens d'arrêt de traitement actionnables par l'utilisateur, telle une touche virtuelle affichée sur l'afficheur 10, qui coopèrent aussi avec les moyens de pilotage pour mettre en pause ou stopper définitivement un traitement par NO en commandant les moyens à valves pour interrompre le passage et la fourniture de mélange NO/N₂ au circuit ventilatoire relié au ventilateur 2.

L'afficheur graphique 10 est, quant à lui, configuré pour afficher la dose de NO à administrer (en 110, 111) avant le début du traitement par NO ou la dose administrée au patient P, pendant le traitement par NO. Les affichages sur l'afficheur graphique 10 sont pilotées par les moyens de pilotage du dispositif de fourniture de NO 1 de l'invention.

Toutefois, selon l'invention, l'afficheur graphique 10 est en outre configuré pour continuer à afficher la dose de NO ayant été administrée 110 au patient, pendant toute une période de pause (dt) durant laquelle le traitement par NO est interrompu temporairement, c'est-à-dire durant une mise en pause qui peut durer plusieurs minutes ou dizaines de minutes, typiquement moins de 45 minutes, par exemple jusqu'à 20 à 30 minutes environ. La mise en pause débute dès l'actionnement par l'utilisateur des moyens d'arrêt de traitement.

Fig. 2 représente un mode de réalisation de l'afficheur graphique 10 du dispositif de fourniture de NO 1 selon l'invention montrant les informations affichées pendant le déroule d'un traitement par NO, par exemple au moyen de l'installation de Fig. 1.

Comme on le voit, pendant le traitement d'un patient par administration de NO, l'afficheur 10 affiche différentes informations utiles au personnel soignant, en particulier :
- la dose de NO à administrer (en 110) au patient, à savoir ici 10 ppm en volume par exemple, qui est choisie avant le début du traitement ;
- la dose de NO réellement administrée (en 111) au patient pendant le traitement par NO, à savoir ici 8 ppm en volume par exemple, c'est-à-dire la dose de NO après mélange du mélange NO/N₂ fourni par le dispositif 1, avec le gaz provenant du ventilateur 2, tel un mélange N₂/O₂ ou de l'air ;
- la quantité de NO₂ formé, à savoir ici 0.1 ppm en volume, présent dans le mélange final administré au patient ; et
- la teneur en oxygène, à savoir ici 25% en volume, présent dans le mélange final administré au patient.

L'afficheur 10 du dispositif 1 affiche aussi d'autres informations, telles des courbes 114 de suivi des teneurs en NO, O₂ et NO₂, pendant le traitement par administration de NO au patient P.

Autrement dit, la dose de NO réglée par l'équipe soignante est visible en permanence (en 110), lorsque l'administration de NO est en cours, et l'afficheur graphique 10 affiche également la dose réellement délivrée (en 111) qui est mesurée par l'analyseur de gaz alimenté par la ligne de prélèvement de gaz 33.

Fig. 3 représente les informations affichées sur l'afficheur graphique 10 du dispositif de fourniture de NO 1 selon l'invention, pendant une période de pause durant laquelle le traitement par NO est interrompu temporairement, c'est-à-dire après une mise en pause.

Dans ce cas, la dose de NO fournie est égale à 0 ppm (en 111) mais conformément à l'invention, l'afficheur graphique 10 continue, malgré la mise en pause, à afficher la dose de NO (en 110) ayant été administrée au patient pendant la phase de traitement, à savoir ici 10 ppmv.

Cet affichage (en 110) est opéré pendant toute la période de pause (dt) durant laquelle le traitement par NO est interrompu temporairement, après actionnement par l'utilisateur de moyens d'arrêt de traitement, tel une touche tactile s'affichant pendant sur l'afficheur graphique 10, tel un écran digital tactile.

Des moyens de démarrage de traitement 120, telle une touche tactile affichée sur l'afficheur graphique 10 permet de reprendre, c'est-à-dire de continuer, le traitement par NO lorsqu'elle est actionnée par l'utilisateur, c'est-à-dire lorsqu'il appuie dessus avec son doigt.

Dans ce cas, le personnel soignant ne risque pas de se tromper lors de la reprise du traitement puisque la dernière dose connue de NO ayant été fournie au patient est affichée en permanence pendant toute la durée de la pause et jusqu'à la reprise du traitement.

Optionnellement, le personnel soignant peut éventuellement la modifier avant de redémarrer le traitement.

## Revendications

1. Dispositif de fourniture de NO (1) comprenant :
- un passage interne pour acheminer un flux gazeux contenant du NO, typiquement un mélange gazeux NO/N₂,
- des moyens à valve pour contrôler le flux gazeux dans le passage interne,
- des moyens de pilotage pour piloter au moins les moyens à valve,
- des moyens de sélection de dose configurés pour permettre à un utilisateur de choisir ou d'ajuster une dose de NO à administrer,
- des moyens de mémorisation configurés pour mémoriser au moins ladite dose de NO à administrer,
- des moyens de démarrage de traitement (120) actionnables par l'utilisateur et coopérant avec les moyens de pilotage pour débuter ou reprendre un traitement par NO,
- des moyens d'arrêt de traitement actionnables par l'utilisateur et coopérant avec les moyens de pilotage pour mettre en pause ou stopper définitivement un traitement par NO et
- un afficheur graphique (10) configuré pour afficher la dose de NO à administrer (110 ; 111) avant le début du traitement par NO ou la dose administrée au patient pendant le traitement par NO,
**caractérisé en ce que** l'afficheur graphique est en outre configuré pour continuer à afficher la dose de NO ayant été administrée (110) au patient, pendant une période de pause (dt) durant laquelle le traitement par NO est interrompu temporairement, en réponse à un actionnement par l'utilisateur des moyens d'arrêt de traitement.

2. Dispositif selon la revendication 1, **caractérisé en ce que** les moyens de pilotage sont configurés pour commander l'afficheur graphique pour afficher la dose de NO à administrer, avant et pendant le traitement par NO et pendant la période de pause (dt) durant laquelle le traitement par NO est interrompu temporairement.

3. Dispositif selon l'une des revendications précédentes, **caractérisé en ce que** les moyens de pilotage sont en outre configurés pour piloter les moyens à valve pour fournir un débit gazeux correspondant à la dose de NO à administrer pendant le traitement par NO, en réponse à l'actionnement par l'utilisateur des moyens de démarrage de traitement.

4. Dispositif selon l'une des revendications précédentes, **caractérisé en ce que** les moyens de pilotage sont en outre configurés pour piloter les moyens à valve pour interrompre temporairement, pendant la période de pause (dt), tout débit gazeux en réponse à l'actionnement par l'utilisateur des moyens d'arrêt de traitement.

5. Dispositif selon l'une des revendications précédentes, **caractérisé en ce que** les moyens de démarrage de traitement (120) et les moyens d'arrêt de traitement comprennent un unique et même organe d'actionnement, actionnable par l'utilisateur.

6. Dispositif selon l'une des revendications 1 à 4, **caractérisé en ce que** les moyens de démarrage de traitement (120) et les moyens d'arrêt de traitement comprennent des organes d'actionnement distincts, actionnables par l'utilisateur.

7. Dispositif selon l'une des revendications 5 ou 6, **caractérisé en ce que** le ou les organes d'actionnement comprennent une ou plusieurs touches virtuelles affichées sur l'afficheur graphique (10).

8. Dispositif selon l'une des revendications précédentes, **caractérisé en ce que** les moyens de pilotage sont configurés pour commander l'afficheur graphique (10) pour continuer à afficher, pendant la période de pause (dt), la dose de NO ayant été administrée (110) au patient juste avant actionnement par l'utilisateur des moyens d'arrêt de traitement, c'est-à-dire avant une mise en pause.

9. Dispositif selon la revendication 8, **caractérisé en ce que** les moyens de mémorisation sont configurés pour mémoriser la dernière dose de NO administrée au patient immédiatement avant le début de la période de pause (dt).

10. Dispositif selon l'une des revendications précédentes, **caractérisé en ce que** la période de pause (dt) a une durée inférieure ou égale à 30 minutes.

11. Dispositif selon la revendication 7, **caractérisé en ce que** la ou les touches virtuelles affichées sur l'afficheur graphique (10) sont des touches tactiles.

12. Dispositif selon l'une des revendications 1 ou 11, **caractérisé en ce que** l'afficheur graphique (10) est à dalle tactile.

13. Installation (100) d'administration de gaz thérapeutique contenant du NO à un patient (P) comprenant un dispositif de fourniture de NO (1) selon l'une des revendications précédentes, alimenté en mélange NO/N₂ par au moins un récipient de gaz sous pression (5) et en oxygène par un récipient d'oxygène sous pression (52), ledit dispositif de fourniture de NO (1) fournissant un mélange NO/N₂ à un circuit de gaz respiratoire (3) raccordé à un ventilateur médical (2) fournissant un mélange O₂/N₂ ou de l'air.

14. Installation selon la revendication 13, **caractérisé en ce que** ledit au moins un récipient de gaz sous pression (5) contient un mélange NO/N₂ contenant de 100 à 1000 ppmv de NO, le reste étant de l'azote.

15. Installation selon la revendication 13, **caractérisé en ce que** le circuit de gaz respiratoire (3) comprend un capteur de débit.

## Patentansprüche

1. Vorrichtung zur Bereitstellung von NO (1), umfassend:
- einen innenliegenden Durchgang zum Befördern eines Gasstroms, der NO enthält, typischerweise ein NO/N₂-Gasgemisch,
- Ventilmittel zum Regeln des Gasflusses in dem innenliegenden Durchgang,
- Steuerungsmittel zum Steuern mindestens der Ventilmittel,
- Mittel zur Dosisauswahl, die so konfiguriert sind, dass der Anwender eine zu verabreichende Dosis von NO auswählen oder anpassen kann,
- Speicherungsmittel, die so konfiguriert sind, dass sie mindestens die genannte zu verabreichende NO-Dosis speichern,
- Mittel zum Starten der Behandlung (120), die von dem Benutzer betätigt werden können und mit den Steuerungsmitteln zusammenwirken, um eine NO-Behandlung zu beginnen oder wieder aufzunehmen,
- Mittel zum Unterbrechen der Behandlung, die von dem Benutzer betätigt werden können und mit den Steuerungsmitteln zusammenwirken, um eine NO-Behandlung zu pausieren oder endgültig abzubrechen, und
- eine grafische Anzeige (10), die so konfiguriert ist, dass sie vor dem Beginn der NO-Behandlung die zu verabreichende NO-Dosis (110; 111) anzeigt oder während der NO-Behandlung die dem Patienten verabreichte Dosis anzeigt,
**dadurch gekennzeichnet, dass** die grafische Anzeige ferner so konfiguriert ist, dass sie die NO-Dosis, die dem Patienten verabreicht (110) wurde, während eines Pausenzeitraums(dt), während dessen die NO-Behandlung vorübergehend unterbrochen ist, als Reaktion auf eine Betätigung der Mittel zum Unterbrechen der Behandlung durch den Benutzer weiterhin anzeigt.

2. Vorrichtung nach Anspruch 1, **dadurch gekennzeichnet, dass** die Steuerungsmittel so konfiguriert ist, dass sie die grafische Anzeige dazu ansteuern, die zu verabreichende NO-Dosis vor und während der NO-Behandlung und während des Pausenzeitraums (dt), während dessen die NO-Behandlung zeitweise unterbrochen ist, anzuzeigen.

3. Vorrichtung nach einem der vorhergehenden Ansprüche, **dadurch gekennzeichnet, dass** die Steuerungsmittel ferner so konfiguriert sind, dass sie als Reaktion auf die Betätigung der Mittel zum Starten der Behandlung durch den Benutzer die Ventilmittel steuern, um einen Gasstrom bereitzustellen, der der während der NO-Behandlung zu verabreichenden NO-Dosis entspricht.

4. Vorrichtung nach einem der vorhergehenden Ansprüche, **dadurch gekennzeichnet, dass** die Steuerungsmittel ferner so konfiguriert sind, dass sie die Ventilmittel steuern, um während des Pausenzeitraums (dt) jeden Gasstrom als Reaktion auf die Betätigung der Mittel zum Unterbrechen der Behandlung durch den Benutzer vorübergehend zu unterbrechen.

5. Vorrichtung nach einem der vorhergehenden Ansprüche, **dadurch gekennzeichnet, dass** die Mittel zum Starten der Behandlung (120) und die Mittel zum Unterbrechen der Behandlung ein einziges, von dem Benutzer betätigbares Betätigungselement umfassen.

6. Vorrichtung nach einem der Ansprüche 1 bis 4, **dadurch gekennzeichnet, dass** die Mittel zum Starten der Behandlung (120) und die Mittel zum Unterbrechen der Behandlung separate Betätigungselemente umfassen, die von dem Benutzer betätigt werden können.

7. Vorrichtung nach einem der Ansprüche 5 oder 6, **dadurch gekennzeichnet, dass** das oder die Betätigungselemente eine oder mehrere virtuelle Tasten umfassen, die auf der grafischen Anzeige (10) angezeigt werden.

8. Vorrichtung nach einem der vorhergehenden Ansprüche, **dadurch gekennzeichnet, dass** die Steuerungsmittel so konfiguriert sind, dass sie die grafische Anzeige (10) so ansteuern, dass sie während des Pausenzeitraums (dt) weiterhin die NO-Dosis anzeigt, die dem Patienten unmittelbar vor der Betätigung der Mittel zum Unterbrechen der Behandlung durch den Benutzer, also vor einer Pause, verabreicht (110) wurde.

9. Vorrichtung nach Anspruch 8, **dadurch gekennzeichnet, dass** die Speicherungsmittel so konfiguriert sind, dass sie die letzte NO-Dosis speichern, die dem Patienten unmittelbar vor Beginn des Pausenzeitraums (dt) verabreicht wurde.

10. Vorrichtung nach einem der vorhergehenden Ansprüche, **dadurch gekennzeichnet, dass** der Pausenzeitraum (dt) eine Dauer von 30 Minuten oder weniger hat.

11. Vorrichtung nach Anspruch 7, **dadurch gekennzeichnet, dass** die virtuellen Tasten, die auf der grafischen Anzeige (10) angezeigt werden, berührungsempfindliche Tasten sind.

12. Vorrichtung nach einem der Ansprüche 1 oder 11, **dadurch gekennzeichnet, dass** die grafische Anzeige (10) ein Touchscreen ist.

13. Anlage (100) zur Verabreichung von NO-haltigem Therapiegas an einen Patienten (P), umfassend eine Vorrichtung (1) zur Bereitstellung von NO nach einem der vorhergehenden Ansprüche, die von mindestens einem Druckgasbehälter (5) mit einem NO/N₂-Gemisch und von einem Drucksauerstoffbehälter (52) mit Sauerstoff versorgt wird, wobei die Vorrichtung (1) zur Bereitstellung von NO ein NO/N₂-Gemisch einem Atemgaskreislauf (3) bereitstellt, der mit einem medizinischen Beatmungsgerät (2) verbunden ist, das ein O²/N₂-Gemisch oder Luft bereitstellt.

14. Anlage nach Anspruch 13, **dadurch gekennzeichnet, dass** der mindestens eine Druckgasbehälter (5) ein NO/N₂-Gemisch enthält, das 100 bis 1000 ppmv NO enthält, wobei der Rest Stickstoff ist.

15. Anlage nach Anspruch 13, **dadurch gekennzeichnet, dass** der Atemgaskreislauf (3) einen Durchflusssensor umfasst.

## Claims

1. NO supply device (1) comprising:
- an internal passage for conveying a gaseous flow containing NO, typically a gaseous mixture NO/N₂,
- valve means for controlling the gaseous flow in the internal passage,
- control means for controlling at least the valve means,
- dose selection means configured to allow a user to choose or adjust an NO dose to be administered,
- memory storage means configured to store at least said NO dose to be administered,
- means (120) for starting treatment, which means are actuatable by the user and cooperate with the control means in order to initiate or resume a treatment by NO,
- means for stopping treatment, which means are actuatable by the user and cooperate with the control means in order to pause or definitively discontinue a treatment by NO, and
- a graphic display (10) configured to display, before the commencement of the treatment by NO, the NO dose to be administered (110; 111), or to display, during the treatment by NO, the dose administered to the patient,
**characterized in that** the graphic display is additionally configured to continue to display the NO dose having been administered (110) to the patient, during a downtime (dt) in which the treatment by NO is interrupted temporarily, in response to an actuation, by the user, of the means for stopping treatment.

2. Device according to Claim 1, **characterized in that** the control means are configured to control the graphic display to display the NO dose to be administered, before and during the treatment by NO and during the downtime (dt) in which the treatment by NO is interrupted temporarily.

3. Device according to one of the preceding claims, **characterized in that** the control means are additionally configured to control the valve means in order to supply a gaseous flowrate corresponding to the NO dose to be administered during the treatment by NO, in response to the actuation, by the user, of the means for starting treatment.

4. Device according to one of the preceding claims, **characterized in that** the control means are additionally configured to control the valve means in order to temporarily interrupt all gaseous flow during the downtime (dt), in response to the actuation, by the user, of the means for stopping treatment.

5. Device according to one of the preceding claims, **characterized in that** the means (120) for starting treatment and the means for stopping treatment comprise one and the same actuation element, which is actuatable by the user.

6. Device according to one of Claims 1 to 4, **characterized in that** the means (120) for starting treatment and the means for stopping treatment comprise separate actuation elements, which are actuatable by the user.

7. Device according to either of Claims 5 and 6, **characterized in that** the one or more actuation elements comprise one or more virtual keys displayed on the graphic display (10).

8. Device according to any one of the preceding claims, **characterized in that** the control means are configured to control the graphic display (10) to continue to display, during the downtime (dt), the NO dose having been administered (110) to the patient just before actuation, by the user, of the means for stopping treatment, that is to say before a pause.

9. Device according to Claim 8, **characterized in that** the memory storage means are configured to store the last dose of NO administered to the patient immediately before the commencement of the downtime (dt).

10. Device according to one of the preceding claims, **characterized in that** the downtime (dt) has a duration of less than or equal to 30 minutes.

11. Device according to Claim 7, **characterized in that** the one or more virtual keys displayed on the graphic display (10) are touch-sensitive keys.

12. Device according to either of Claims 1 and 11, **characterized in that** the graphic display (10) is a touch panel.

13. Installation (100) for administering therapeutic gas containing NO to a patient (P), comprising an NO supply device (1) according to one of the preceding claims, supplied with NO/N₂ mixture by at least one pressurized gas container (5) and with oxygen by a pressurized oxygen container (52), said NO supply device (1) supplying an NO/N₂ mixture to a respiratory gas circuit (3) connected to a medical ventilator (2) supplying an O₂/N₂ mixture or air.

14. Installation according to Claim 13, **characterized in that** said at least one pressurized gas container (5) contains an NO/N₂ mixture containing from 100 to 1000 ppmv of NO, the remainder being nitrogen.

15. Installation according to Claim 13, **characterized in that** the respiratory gas circuit (3) comprises a flowrate sensor.
